(19) Europäisches Patentamt
European Patent Office
Office européen des brevets

(11) Publication number : **0 687 468 A2**

(12) # EUROPEAN PATENT APPLICATION

(21) Application number : **95109281.6**

(51) Int. Cl.[6] : **A61K 31/275**

(22) Date of filing : **15.06.95**

(30) Priority : **16.06.94 JP 134445/94**

(43) Date of publication of application : **20.12.95 Bulletin 95/51**

(84) Designated Contracting States : **AT BE CH DE DK ES FR GB GR IE IT LI LU MC NL PT SE**

(71) Applicant : **Toyo Boseki Kabushiki Kaisha No.2-8, Dojimahama 2-chome Kita-ku Osaka-shi Osaka 530 (JP)**

(72) Inventor : **Watanabe, Akihiko c/o Toyo Boseki K.K. Res. Inst. 1-1 Katata 2-chome Ohtsu-shi, Shiga 520-02 (JP)**
Inventor : **Tominaga, Takanari c/o Toyo Boseki K.K. Res. Inst. 1-1 Katata 2-chome Ohtsu-shi, Shiga 520-02 (JP)**
Inventor : **Taguchi, Hiroaki c/o Toyo Boseki K.K. Res. Inst. 1-1 Katata 2-chome Ohtsu-shi, Shiga 520-02 (JP)**

(74) Representative : **von Kreisler, Alek, Dipl.-Chem. Patentanwälte, von Kreisler-Selting-Werner, Bahnhofsvorplatz 1 (Deichmannhaus) D-50667 Köln (DE)**

(54) **Pharmaceutical composition for suppressing infiltration of eosinophils**

(57) A pharmaceutical composition for suppressing infiltration of eosinophils, comprising, as an active ingredient, a benzonitrile derivative of the formula (I)

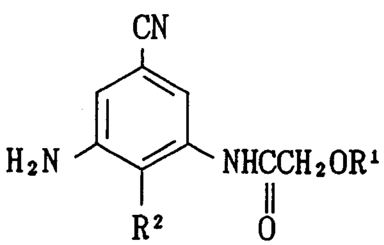

(I)

wherein $R^1$ is a hydrogen atom, a lower alkyl having 1 to 6 carbon atoms or an acyl having 2 to 10 carbon atoms, and $R^2$ is a hydrogen atom or a halogen atom, and a method for suppressing infiltration of eosinophils, comprising administering said benzonitrile derivative. The compound of the formula (I) inhibits adhesion of eosinophils to vascular endothelial cells and strongly suppresses infiltration of eosinophils into inflammatory lesions. Accordingly, the compound of the formula (I) can be used as an antiinflammatory agent for the prophylaxis and treatment of various diseases associated with infiltration of eosinophils.

EP 0 687 468 A2

## FIELD OF THE INVENTION

The present invention relates to a pharmaceutical composition for suppressing infiltration of eosinophils, which comprises a benzonitrile derivative as an active ingredient. The benzonitrile derivative which is the active ingredient in the present invention inhibits adhesion of eosinophils to vascular endothelial cells and suppresses infiltration of eosinophils into inflammatory lesions, and is useful for the prophylaxis and treatment of diseases associated with infiltration of eosinophils into inflammatory lesions, such as eczema, pemphigus, pemphoid, dermatitis herpetiformis, psoriasis, chronic obstructive pulmonary disease, hypersensitive pneumonitis, bronchopulmonary aspergillosis, eosinophilic pneumonia, eosinophilic enterogastritis and colitis ulcerosa.

The present invention also relates to a method for suppressing infiltration of eosinophils, comprising administering said benzonitrile derivative.

## BACKGROUND OF THE INVENTION

As a compound which has an inhibitory action on the adhesion of eosinophils to vascular endothelial cells and suppresses infiltration of eosinophils into inflammatory lesions, for example, an anti-VLA-4 antibody is described in Journal of Experimental Medicine, *177*, P. 561 (1993).

However, said anti-VLA-4 antibody is a polypeptide which cannot show effects by oral administration, and the administration route is limited to an injection such as intravenous administration, burdening patients with pain.

The compound of the formula (I) of the present invention is described in Japanese Patent Unexamined Publication No. 311450/1990. Yet, the publication does not disclose an inhibitory action against adhesion of eosinophils or a suppressive action on the infiltration of eosinophils.

It has been clarified in recent years that infiltration of eosinophils into inflammatory lesions causes symptoms such as eczema, pemphigus, pemphoid, dermatitis herpetiformis, psoriasis, chronic obstructive pulmonary disease, hypersensitive pneumonitis, bronchopulmonary aspergillosis, eosinophilic pneumonia, eosinophilic enterogastritis and colitis ulcerosa, as well as aggravates inflammation. It has also been clarified that adhesion of eosinophils to vascular endothelial cells is an important phase in the infiltration of eosinophils into inflammatory lesions.

Therefore, a pharmaceutical agent capable of inhibiting the adhesion of eosinophils to vascular endothelial cells suppresses infiltration of eosinophils into inflammatory lesions and is effective for suppressing the abovementioned inflammations which proceed along with the infiltration of eosinophils.

## SUMMARY OF THE INVENTION

Accordingly, it is an object of the present invention to provide a pharmaceutical composition which permits oral administration and effectively suppresses infiltration of eosinophils into inflammatory; lesions by inhibiting adhesion of eosinophils to vascular endothelial cells.

Another object of the present invention is to provide a method for suppressing infiltration of eosinophils.

The present inventors have intensively studfed to provide a pharmaceutical composition for suppressing infiltration of eosinophils, which permits oral administration and is superior in therapeutic effects against the symptoms caused by infiltration of eosinophils into the inflammatory lesions, and found that a certain benzonitrile derivative strongly inhibits the adhesion of eosinophils to vascular endothelial cells and that the derivative is superior in the suppression of infiltration of eosinophils into the inflammatory lesions, which resulted in the completion of the present invention.

## DETAILED DESCRIPTION OF THE INVENTION

The present invention relates to the following (1) to (5).

(1) A pharmaceutical composition for suppressing infiltration of eosinophils, comprising, as an active ingredient, a benzonitrile derivative of the formula (I)

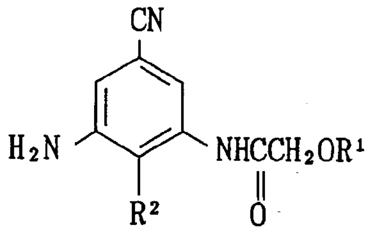

(I)

wherein $R^1$ is a hydrogen atom, a lower alkyl having 1 to 6 carbon atoms or an acyl having 2 to 10 carbon atoms, and $R^2$ is a hydrogen atom or a halogen atom, or a pharmacologically acceptable acid addition salt thereof [hereinafter also referred to as benzonitrile derivative (I)].

(2) A method for suppressing infiltration of eosinophils, comprising administering a pharmaceutically effective amount of a benzonitrile derivative (I).

(3) A method for the prophylaxis and treatment of the diseases associated with infiltration of eosinophils, comprising administering a pharmaceutically effective amount of a benzonitrile derivative (I).

(4) Use of a benzonitrile derivative (I) for producing a pharmaceutical composition for suppressing infiltration of eosinophils.

(5) Use of a benzonitrile derivative (I) for producing a pharmaceutical composition for the prophylaxis and treatment of diseases associated with infiltration of eosinophils.

In the formula (I), the lower alkyl having 1 to 6 carbon atoms, which is represented by $R^1$ includes, for example, methyl, ethyl, n-propyl, n-butyl, n-pentyl, n-hexyl and isomers thereof (e.g. isopropyl, isobutyl, sec-butyl, tert-butyl, isopentyl and neopentyl), with preference given to methyl and ethyl.

In the formula (I), the acyl having 2 to 10 carbon atoms, which is represented by $R^1$ includes, for example, acetyl, propionyl, butyryl, isobutyryl, valeryl, isovaleryl, pivaloyl, hexanoyl, heptanoyl, octanoyl, nonanoyl, decanoyl, cyclopentylcarbonyl, cyclohexylcarbonyl, oxalo, carboxyacetyl, 3-carboxypropionyl, benzoyl and substituted benzoyl, with preference given to acetyl, propionyl, butyryl, isobutyryl and benzoyl.

In the formula (I), the halogen atom which is represented by $R^2$ includes, for example, fluorine atom, chlorine atom, bromine atom and iodine atom, with preference given to chlorine atom.

In the present invention, the compound of the formula (I) optionally forms a salt with a pharmacologically acceptable acid, and the present invention encompasses the use of such acid addition salt.

Examples of the acid addition salt include salts with inorganic acid, such as hydrochloride, hydrobromide, hydroiodide, sulfate, phosphate, nitrate, and salts with organic acid, such as acetate, lactate, citrate, methanesulfonate, ethanesulfonate, benzenesulfonate, toluenesulfonate and isethionate.

In the present invention, the compound of the formula (I) can be produced by reacting one mole of a diamino compound of the formula (II)

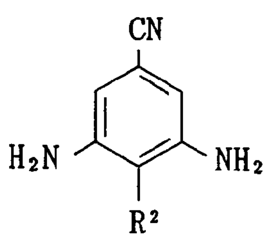

(II)

wherein $R^2$ is a hydrogen atom or a halogen atom, with one mole of an acid chloride of the formula (III)

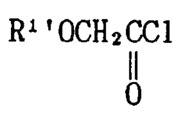

(III)

wherein $R^{1'}$ is a lower alkyl having 1 to 6 carbon atoms or an acyl having 2 to 10 carbon atoms.

The reaction is carried in a solvent such as pyridine, chloroform, dichloromethane, diethyl ether and N,N-dimethylformamide, in the presence of a base such as pyridine and triethylamine at -30°C to the refluxing temperature of the solvent used.

The reaction can be also carried out in an aqueous alkaline solution as in the Schotten Baumann method.

With regard to the present invention, the compound of the formula (I) wherein $R^1$ is a hydrogen atom can be obtained by hydrolysis of a compound of the formula (I) wherein $R^1$ is an acyl having 2 to 10 carbon atoms.

The compound of the formula (I) can be converted to an acid addition salt by a conventional method.

The representative compounds of the formula (I) are as follows.

3-(acetoxyacetylamino)-5-amino-4-chlorobenzonitrile
3-amino-4-chloro-5-(methoxyacetylamino)benzonitrile
3-amino-4-chloro-5-(hydroxyacetylamino)benzonitrile

The compound of the formula (I) which is the active ingredient in the present invention inhibits the adhesion of eosinophils to vascular endothelial cells and exhibits strong suppressive action on the infiltration of eosinophils into inflammatory lesions. Hence, the compound shows an antiinflammatory action against inflammations which proceed along with the infiltration of eosinophils, and is useful as an antiinflammatory agent for the prophylaxis and treatment of various diseases associated with the infiltration of eosinophils. Examples of such diseases include eczema, pemphigus, pemphoid, dermatitis herpetiformis, psoriasis, chronic obstructive pulmonary disease, hypersensitive pneumonitis, bronchopulmonary aspergillosis, eosinophilic pneumonia, eosinophilic enterogastritis and colitis ulcerosa.

The pharmaceutical composition of the present invention is administered to mammals inclusive of human by an oral or parenteral route or by inhalation. When in use, the compound is prepared into various preparation forms by a conventional method, using pharmaceutically acceptable excepients. For example, tablet, pill, capsule, granule, powder or liquid preparation is generally used for oral administration. Injection, suppository or dermatologic preparation is generally used for parenteral administration. Alternatively, the preparation can be transpulmonarily administered by an aerosol spray or by inhalation of a dry powder.

Examples of the pharmaceutically acceptable excipient include water, physiological saline, vegetable oil, ethanol, polyethylene glycol, glucose, lactose, mannitol, sucrose, talc, starch, magnesium silicate, magnesium stearate, gum arabic, gelatin, hydroxypropylcellulose, hydroxypropylmethylcellulose, polyvinylpyrrolidone, lanolin and petrolatum.

While the dose of the compound of the formula (I) varies depending on the age, sex, symptom, the kind of disease and administration route, it is generally 1 mg to 900 mg daily for an adult, which is administered in a single to several doses a day.

The inhibitory activity of the compound of the formula (I) against adhesion of eosinophils to vascular endothelial cells and suppressive activity thereof on the infiltration of eosinophils into inflammatory lesions are shown in the following Examples.

Example 1 Inhibition of adhesion of eosinophils

Human umbilical vein-derived endothelial cells (HUVEC) were placed in a single layer in a 96 well plate and treated with 100 U/ml tumor necrosis factor (TNF) for 6 hours. The eosinophils obtained from peripheral blood of healthy human were fluorescence-labeled and layered on the TNF-treated HUVEC. After adhesion reaction for 20 minutes, unadhered eosinophils were removed by washing and fluorescent intensity of each well of the plate was measured to determine the eosinophils adhered to the HUVEC. The test compound was added so that the concentration at TNF treatment and adhesion reaction was $10^{-6}$M. The results are shown in Table 1.

Table 1

| Test compound | Suppression of adhesion of eosinophils (%) |
|---|---|
| 3-(acetoxyacetylamino)-5-amino-4-chlorobenzonitrile | 70 |
| 3-amino-4-chloro-5-(methoxyacetylamino)benzonitrile | 62 |
| 3-amino-4-chloro-5-(hydroxyacetylamino)benzonitrile | 74 |

Example 2 Intraperitoneal infiltration of eosinophils in rat

Ascaris extract (Asc, protein amount 8 mg) was intraperitoneally administered to male Wistar rats to sensitize them. Seven days later, 4 mg protein amount of Asc was intraperitoneally administered to induce intraperitoneal infiltration of eosinophils. Forty-eight hours after the antigen induction, abdominal cavity was washed, and the infiltrated eosinophils were counted. The test compound (3 mg/kg) was orally administered once a day for 9 days from the day of antigen sensitization to one day before recovery of the infiltrated intraperitoneal cells. The results are shown in Table 2.

Table 2

| Test compound | Suppression of infiltration of eosinophils (%) |
|---|---|
| 3-(acetoxyacetylamino)-5-amino-4-chlorobenzonitrile | 55 |
| 3-amino-4-chloro-5-(methoxyacetylamino)benzonitrile | 42 |
| 3-amino-4-chloro-5-(hydroxyacetylamino)benzonitrile | 54 |

Example 3 Infiltration of eosinophils into bronchovesicles in rats

An ovalbumin (OA) antigen (10 mg/kg) was intramuscularly administered to male BN rats, and $5 \times 10^{10}$ nonvial *Bordetella pertussis* cells were intraperitoneally administered to sensitize them. Fourteen days later, the rats were exposed to 2% OA to induce infiltration of eosinophils in the airway. Twenty-four hours later, bronchial tubes were washed and infiltrated cells were recovered to count the infiltrated eosinophils. The test compound (10 mg/kg) was orally administered 5 minutes before and 5 hours after the induction with antigen. The results are shown in Table 3.

Table 3

| Test compound | Suppression of infiltration of eosinophils (%) |
|---|---|
| 3-(acetoxyacetylamino)-5-amino-4-chlorobenzonitrile | 53 |
| 3-amino-4-chloro-5-(methoxyacetylamino)benzonitrile | 56 |
| 3-amino-4-chloro-5-(hydroxyacetylamino)benzonitrile | 48 |

Formulation Example 1

A surfactant, Span 85 (0.1 g, trademark), was dissolved in a chlorofluorocarbon solvent, Freon 11 (5.34 g, trademark). 3-(Acetoxyacetylamino)-5-amino-4-chlorobenzonitrile (0.2 g) finely divided into particles having an average particle size of about 2 μm by a jet mill was dispersed therein, and the dispersion was sealed in an aluminum container, which was then equipped with a valve. Then, chlorofluorocarbon, Freon 12 (1.49 g, trademark), was filled in the container to give an aerosol.

Formulation Example 2

3-(Acetoxyacetylamino)-5-amino-4-chlorobenznitrile (600 g), lactose (1,000 g) and corn starch (600 g) were granulated by a fluidized bed granulator using a binder solution containing polyvinylpyrrolidone (30 g) dissolved in water (1190 ml). The granules (100 g) and magnesium stearate (1 g) were mixed and the mixture (180 mg) was compressed using a 8 mm diameter die and punch to give tablets.

## Claims

1. Use of a benzonitrile derivative of the formula (I)

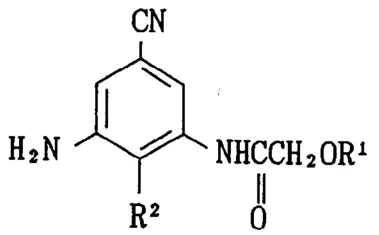

(I)

wherein $R^1$ is a hydrogen atom, a lower alkyl having 1 to 6 carbon atoms or an acyl having 2 to 10 carbon atoms, and $R^2$ is a hydrogen atom or a halogen atom, or a pharmacologically acceptable acid addition salt thereof, for producing a pharmaceutical composition for suppressing infiltration of eosinophils.

2. The use of Claim 1 wherein $R^1$ is a hydrogen atom.

3. The use of Claim 1 wherein $R^1$ is methyl or ethyl.

4. The use of Claim 1 wherein $R^1$ is acetyl, propionyl, butyryl, isobutyryl or benzoyl.

5. The use of Claim 1 wherein $R^2$ is a chlorine atom.

6. Use of the benzonitrile derivative of Claim 1 or a pharmacologically acceptable acid addition salt thereof for producing a pharmaceutical composition for the prophylaxis or treatment of the diseases associated with infiltration of eosinophils.